# EUROPÄISCHE PATENTSCHRIFT

(11) **EP 2 451 415 B1**
(45) Veröffentlichungstag und Bekanntmachung des Hinweises auf die Patenterteilung: **28.03.2018**
(21) Anmeldenummer: 09777095.2
(22) Anmeldetag: 10.07.2009
(51) Int. Cl.: A61F 9/008, A61F 9/009

(54) **VORRICHTUNG ZUM SCHNEIDEN EINES GEWEBETEILS EINES AUGES MITTELS LASERSTRAHLUNG**
DEVICE FOR CUTTING A TISSUE PART OF AN EYE BY MEANS OF LASER RADIATION
DISPOSITIF POUR DÉCOUPER UN LAMBEAU DE TISSU OCULAIRE AU MOYEN D UN FAISCEAU LASER

(43) Veröffentlichungstag der Anmeldung: 16.05.2012
(73) Patentinhaber: WaveLight GmbH, 91058 Erlangen (DE)
(72) Erfinder: DONITZKY, Christof, 90542 Eckental/Eschenau (DE); DEISINGER, Thomas, 90556 Cadolzburg (DE); ZERL, Bernd, 91080 Uttenreuth (DE)
(74) Vertreter: von Hellfeld, Axel
(86) Internationale Anmeldenummer: PCT/EP2009/005011
(87) Internationale Veröffentlichungsnummer: WO 2011/003431

(56) Entgegenhaltungen:
- EP-A1- 1 970 034
- US-A1- 2007 093 795
- US-A1- 2008 071 254

## Beschreibung

Die Erfindung betrifft eine Vorrichtung zum Schneiden eines Gewebeteils eines Auges mit den Merkmalen des Oberbegriffs des Patentanspruchs 1. Eine solche Vorrichtung ist aus der PCT/EP2008/006962 bekannt.

Die Erfindung befasst sich allgemein mit der Herstellung einer mechanischen Kopplung zwischen einem biologischen Gewebe und einer Lasereinrichtung, deren Laserstrahlung zur Behandlung des Gewebes genutzt wird. Im speziellen befasst sich die Erfindung mit der Ankopplung einer Lasereinrichtung an ein Auge, insbesondere ein menschliches Auges, um mit der Laserstrahlung einen oder mehrere Schnitte in das Auge einzubringen.

Für eine gezielte Wirkung der Laserstrahlung ist eine präzise Lokalisierung des Strahlfokus relativ zu dem zu behandelnden Gewebe unerlässlich. Insbesondere für die Erzeugung von Schnitten im Gewebe wird häufig ein verhältnismäßig kleiner Strahlfokus angestrebt, um die Schnittdicke gering zu halten. Entsprechend präzise muss dann auch die Positionierung des Strahlfokus sein. Dies gilt insbesondere für die Einbringung von Schnitten in Augengewebe, wie sie beispielsweise im Rahmen der sogenannten Fs-LASIK vorkommen. LASIK steht für Laser In-situ Keratomileusis und bezeichnet eine Technik zur Behandlung von Fehlsichtigkeiten, bei der zunächst aus dem vorderen Bereich der Kornea ein Deckelscheibchen (in der Fachwelt regelmäßig als Flap bezeichnet) herausgeschnitten wird, das zum Teil noch mit der Kornea verbunden bleibt, so dass es für eine anschließende Ablation von darunter liegendem Korneagewebe mittels Laserstrahlung zur Seite geklappt werden kann. Nach Durchführung der Ablation (Gewebeabtrag) wird der Flap zurückgeklappt und es erfolgt eine relative schnelle Heilung unter weitgehender Unversehrtheit der Korneaoberfläche.

Für die Erzeugung des Flaps verwendet eine bisher gängige Technik einen mechanischen Schneidhobel (Mikrokeratom), welcher mit einer schnell oszillierenden Schneide von der Seite her in die Kornea einschneidet. Es wird seit einiger Zeit auch an Systemen gearbeitet, die eine Flaperzeugung mittels fokussierter Laserstrahlung mit Pulsdauern im Femtosekundenbereich gestatten. Daher der Name Femtosekunden- oder Fs-LASIK. Die Strahlung wird dabei unter der Vorderfläche der Kornea im Inneren des Gewebes fokussiert und die Fokuspunkte werden in der gewünschten Fläche so positioniert, dass im Ergebnis ein Flap aus der Kornea herausgeschnitten wird. Gewebeschnitte im Auge werden aber nicht nur bei der Fs-LASIK benötigt, sondern auch bei anderen Indikationen, etwa bei der Keratoplastik (z.B. anteriore oder posteriore lamellare Keratoplastik, penetrierende Keratoplastik bei Hornhauttransplantationen), der Fs-Lentikel-Extraktion zur Refraktionskorrektur, beim Schneiden von interkornealen Ringsegmenten zur Stabilisierung von Keratokonus und Hornhautvorwölbung (z.B. für das Einsetzen von Intacs, d.h. kleinen implantierten Ringsegmenten zur biomechanischen Stabilisierung der Hornhaut), bei Kataraktinzisionen, bei Presbyopie-Schnitten in der Augenlinse, bei intrastromalen Inlays, bei der Keratomie für Astigmatismen, bei der kornealen Resektion und dgl.

Es ist im Stand der Technik bekannt (siehe z.B. EP 1970034, US 2008 071254, US 5,549,632, WO 03/002008 A1), bei Augenlasereinrichtungen eine planparallele Applanationslinse auf die Kornea aufzudrücken. Durch das Aufdrücken der Applanationslinse verformt sich das Auge und schmiegt sich flächig an die augenzugewandte Unterseite der Applanationslinse an. Der Strahlfokus der Laserstrahlung ist in z-Richtung gegenüber der Applanationslinse referenziert (mit z-Richtung ist hierbei die Strahllängsrichtung gemeint). Durch das Anliegen des Auges an der Applanationslinse ist ein fester z-Bezug zwischen Auge und Linse gegeben, was eine präzise z-Positionierung des Strahlfokus in beliebigen Regionen in der Kornea oder anderen Gewebestrukturen in der Tiefe des Auges gestattet.

Neben planparallelen Applanationslinsen sind im Stand der Technik auch Linsen (oder allgemein Kontaktgläser) mit sphärisch, asphärisch oder anderweitig gekrümmten Flächen bekannt geworden, die gleichermaßen eine z-Referenzierung ermöglichen. Durch geeignet konkave Gestaltung der augenzugewandten Linsenunterseite kann die Verformung des Auges beim Aufsetzen der Linse verringert werden. Dies ist insofern vorteilhaft, als sich der Augeninnendruck nicht so sehr erhöht wie bei einer aufgedrückten Applanationslinse mit planer Unterseite. Allerdings verschlechtern die gekrümmten Linsenflächen die Fokussierbarkeit der Laserstrahlung.

Um das Auge des Patienten in fester Distanz zur Fokussieroptik der Lasereinrichtung zu halten, werden im Stand der Technik in der Regel Saugringe verwendet, welche mittels Unterdruck an die Sklera des Auges angesaugt werden und die Kornea ringförmig umgeben. Entweder ist die Applanationslinse dabei in den Saugring integriert, wie beispielsweise in Fig. 4C der oben erwähnten US 5,549,632 gezeigt. Oder es ist die Applanationslinse Teil einer gesonderten Komponente, welche mit dem Saugring gekoppelt wird, siehe beispielsweise Fig. 7 von WO 03/002008 A1, wo die Applanationslinse fest an einem Kegelkörper angebracht ist, der im Bereich seiner Kegelbasis zur Ankopplung an die Fokussieroptik einer Lasereinrichtung ausgeführt ist und an seinem schmalen Kegelende mittels einer gesonderten Klemmzange in feste Kopplung mit dem Saugring gebracht werden kann.

Die dreiteilige Konstruktion gemäß WO 03/002008 A1 mit einem Saugring, einer Klemmzange und einem die Applanationslinse tragenden Kegelkörper gestattet eine voneinander unabhängige Heranführung der Bearbeitungsoptik der Lasereinrichtung und des Patientenauges bis nahe aneinander. Der Saugring sitzt dabei schon auf dem Auge, während der Kegel schon an der Bearbeitungsoptik angebracht ist. Sind die Bearbeitungsoptik und das Patientenauge nahe genug aneinander herangebracht, kommt die Klemmzange zum Einsatz, welche die mechanische Kopplung zwischen dem Kegel und dem Saugring herstellt.

Es ist leicht nachvollziehbar, dass die mechanische Fixierung des Auges gegenüber der Bearbeitungsoptik mittels der Klemmzange eine kritische Phase der Operationsvorbereitung ist. Die dabei wirkenden Druck- und Scherkräfte dürfen am Patientenauge nicht zu Verletzungen führen oder durch ungenaue Positionierung der beteiligten mechanischen Komponenten zueinander eine übermäßige und möglicherweise gefährliche Erhöhung des Augeninnendrucks bewirken. Tritt über einen längeren Zeitraum ein erhöhter Augeninnendruck auf, kann dies unter Umständen zu einer Schädigung des Sehnervs führen. Selbst wenn es möglich ist, die Optik mittels eines Steuerknüppels (Joystick) mehr oder weniger präzise zu positionieren, ist der Kegel dennoch starr mit der Optik verbunden, weswegen der letztendlich herbeigeführte mechanische Kontakt zum Auge und die damit verbundene Einebnung des Auges starr bleiben. Die hierbei auf das Auge ausgeübten Kräfte, und zwar sowohl die beim Vorgang der Ankopplung auftretenden Kräfte als auch die nach erfolgter Applanation wirkenden Kräfte, sind kaum vorhersehbar und können von Patient zu Patient unterschiedlich sein.

Insgesamt ist für zahlreiche im Stand der Technik bekannte Lösungen charakteristisch, dass eine Applanationslinse, oder allgemeiner ausgedrückt, ein Kontaktglas, gleich welcher Form, im Zuge der Ankopplung des Auges an die Bearbeitungsoptik auf das Auge aufgedrückt wird, und zwar so stark, dass sich das Auge in dem für die nachfolgende Bearbeitung erforderlichen Bereich an das Kontaktglas anschmiegt. Dieses Aufdrücken des Kontaktglases auf das Auge ist regelmäßig mit Druckstößen verbunden, die vom Patienten als unangenehm empfunden werden können. Das Auge wird dabei gequetscht und kann unter Umständen Schaden nehmen, insbesondere weil die auftretenden Zug-, Druck- und Scherkräfte nicht genau vorherbestimmbar sind.

Der Erfindung liegt die Aufgabe zugrunde, eine Vorrichtung der eingangs genannten Art so auszugestalten, dass eine optimale Kopplung des Patientenauges an die Optik des Lasersystems gefördert ist. Unerwünschte Belastungen des Auges wie insbesondere Gewebeeindrückungen oder Verletzungen des Epithels sollen verhindert werden.

Hierzu sieht die Erfindung eine Vorrichtung zum Schneiden eines Gewebeteils eines Auges mittels fokussierter Laserstrahlung vor, die folgende Elemente umfasst:
- eine auf das Auge aufsetzbare Saugringeinheit mit einer Ringachse,
- eine von der Saugringeinheit gesonderte, längs der Ringachse in Koppelkontakt mit dieser bewegbare mechanischen Schnittstelleneinheit, die mit optischen Mitteln mechanisch koppelbar ist, welche die Laserstrahlung auf oder in das Gewebeteil des Auges fokussieren, und
- Dichtmittel, die bei Bewegung der Schnittstelleneinheit in Koppelkontakt mit der Saugringeinheit einen evakuierbaren Raum bilden, der von Dichtflächen der Schnittstelleneinheit und der Saugringeinheit und den Dichtmitteln abgegrenzt ist.

Der vorstehend genannte evakuierbare Raum wird also erfindungsgemäß ausschließlich durch mechanische Komponenten der Vorrichtung selbst abgegrenzt und nicht durch Teile des Auges. Dies bedeutet, dass die im Stand der Technik noch vielfach vorgesehene Fixierung der Schnittstelleneinheit durch Vakuum direkt an der Kornea erfindungsgemäß nicht stattfindet sondern vielmehr in einem ersten Schritt der Ankopplung nur die mechanischen Komponenten "Schnittstelleneinheit" und "Saugringeinheit" miteinander mechanisch fest verkoppelt werden, und zwar durch Erzeugung eines Vakuums in dem genannten evakuierbaren Raum, der ausschließlich durch Flächen abgegrenzt ist, die entweder zur Schnittstelleneinheit oder zur Saugringeinheit gehören, nicht aber zum Auge.

Gemäß einer bevorzugten Ausgestaltung sind die Dichtflächen der Saugringeinheit und die hierzu komplementären Dichtflächen der Schnittstelleneinheit jeweils kegelstumpfförmig (konisch).

Die Dichtmittel können im entkoppelten Zustand, also in einem Zustand, in dem die Saugringeinheit noch von der Schnittstelleneinheit entfernt ist, an der Saugringeinheit und/oder der Schnittstelleneinheit vorgesehen sein.

Bevorzugt sind die Dichtmittel jeweils ringförmig und aus elastischem Material. Es ist auch möglich, die Dichtung durch einen mechanischen Passsitz selbstdichtend zu erzeugen.

Nachfolgend wird ein Ausführungsbeispiel der Erfindung anhand der Zeichnung näher beschrieben. Es stellen dar:
Fig. 1a-1c schematisch aufeinanderfolgende Phasen beim Ankoppeln einer Schnittstelleineinheit an eine auf einem Auge sitzende Saugringeinheit; und
Fig. 2 schematisiert ein Ausführungsbeispiel einer Lasereinrichtung zum Schneiden eines Gewebeteils eines Auges.

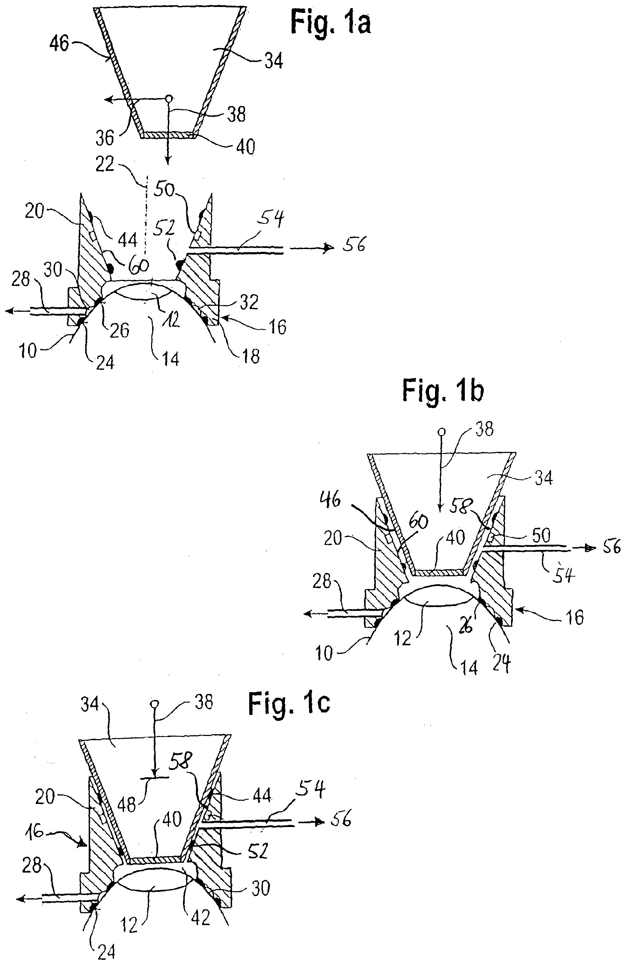

Bei dem in den Figuren 1a-1c gezeigten Ausführungsbeispiel ist das zu behandelnde (menschliche) Auge mit 10 bezeichnet. Die Kornea (Hornhaut) des Auges 10 ist bei 12 gezeigt, während die Sclera (Lederhaut) mit 14 bezeichnet ist.

In die Kornea 12 des Auges 10 sollen mittels gepulster Laserstrahlung mit Pulsdauern im Femtosekundenbereich ein oder mehrere Schnitte eingebracht werden. Die hierfür notwendige Laserstrahlung wird von einer nicht näher dargestellten Laserquelle bereitgestellt. Beispielsweise liegt die Wellenlänge der in das Auge 10 eingestrahlten Behandlungsstrahlung im nahen Infrarotbereich. Beispielsweise kann ein bei 1030 nm emittierender Yb-Laser verwendet werden.

Bevor mit der Laserbehandlung des Auges begonnen wird, ist zunächst das Auge 10 an die mit der Laserquelle ausgestattete Lasereinrichtung anzukoppeln, um den Strahlfokus präzise in der sogenannten z-Richtung (Strahlungsrichtung) in der Kornea positionieren zu können. Hierfür wird zunächst eine Saugringeinheit 16 in an sich bekannter Weise auf das Auge 10 aufgesetzt und durch Unterdruck an dem Auge 10 fixiert. Die Saugringeinheit 16 stabilisiert und fixiert das Auge 10. Sie weist ein den eigentlichen Saugring bildendes Unterteil 18, einen an das Unterteil 18 anschließenden, einstückig mit diesem hergestellten Einführtrichter 20 sowie eine Ringachse 22 auf. Das Unterteil 18 bildet zwei jeweils zur Anlage an der Sclera 14 bestimmte, ringförmig umlaufende Dichtflächen 24, 26, welche zwischen sich eine mit einem Evakuierungskanal 28 verbundene, ringförmig umlaufende Saugkammer 30 begrenzen. Die Dichtflächen 24, 26 können beispielsweise jeweils von einem gesonderten, am Untereil 18 angebrachten Dichtelement gebildet sein. Zur Bildung der Saugkammer 30 ist am augenzugewandten Innenumfangsmantel des Unterteils 18 eine entsprechende Ringnut - bezeichnet mit 32 - gebildet. Die Saugkammer 30 ist ausschließlich zwischen der Saugringeinheit 16 und der Sclera 14 begrenzt. Durch Evakuierung der Saugkammer 30 saugt sich die Saugringeinheit 16 am Auge 10 fest. Hierzu wird der Evakuierungskanal 28 mit einer nicht näher dargestellten Unterdruckquelle in Form einer Evakuierungspumpe verbunden.

Mechanisches Gegenstück zu der solchermaßen auf dem Auge 10 fixierten Saugringeinheit 16 ist eine allgemein mit 34 bezeichnete Schnittstelleneinheit, welche in nicht näher dargestellter Weise fest, jedoch lösbar mit einer Fokussieroptik der erwähnten Lasereinrichtung koppelbar ist. Die Schnittstelleneinheit 34 ist zusammen mit der Fokussieroptik entlang einer durch einen Horizontalpfeil 36 angegebenen Horizontalrichtung und einer durch einen Vertikalpfeil 38 angedeuteten Vertikalrichtung relativ zu dem Patienten und der an ihm befestigten Saugringeinheit 16 verfahrbar. Die Verfahrbarkeit der Schnittstelleneinheit 34 kann zumindest teilweise motorisch bewirkt sein, beispielsweise mittels eines elektromotorischen Antriebs. Auch eine wenigstens teilweise händische Bewegbarkeit der Schnittstelleneinheit 34 relativ zu der Saugringeinheit 16 ist vorstellbar.

Insgesamt ist die Schnittstelleneinheit 34 kegelstumpfförmig ausgebildet, wobei sie an ihrem breiteren Kegelende (in den Figuren 1a-1c obenliegend) zur Kopplung mit der Fokussieroptik ausgebildet ist und an ihrem schmäleren Kegelende ein im gezeigten Beispielfall als planparallele Applanationslinse ausgebildetes Kontaktglas 40 trägt.

In einer ersten Phase des Ankoppelvorgangs des Auges 10 an die Lasereinrichtung wird die Schnittstelleneinheit 34 relativ zu der Saugringeinheit 16 in Pfeilrichtung 36 in eine Position bewegt, in der sie koaxial über dem Einführtrichter 20 steht, so dass die Schnittstelleneinheit 34 anschließend durch axiales Absenken in den Einführtrichter 20 eintauchen kann. Die Phase des Eintauchens der Schnittstelleneinheit 34 in den Einführtrichter 20 der Saugringeinheit 16 ist in Fig. 1b dargestellt. Beim Absenken der Schnittstelleneinheit 34 nähert sich die Applanationslinse 40 dem Auge 10; gleichzeitig wird der radiale Luftspalt zwischen dem Einführtrichter 20 und der Schnittstelleneinheit 34 kleiner. Der konusförmig verlaufende Innenumfangsmantel 60 des Einführtrichters 20 und der gleichermaßen konusförmig, d.h. komplementär verlaufende Außenumfangsmantel 46 der Schnittstelleneinheit 34 tragen oder bilden jeweils Dichtmittel 44, 52.

Die zwischen der Saugringeinheit 16 und der Schnittstelleneinheit 34 wirksamen Dichtmittel sind im gezeigten Beispielfall von einer an dem Einführtrichter 20 angebrachten Ringdichtung 44 und dem dieser Ringdichtung 44 im eingetauchten Zustand gegenüberliegenden Teil des Außenumfangsmantels der Schnittstelleneinheit 34 gebildet. Dieser als Dichtfläche wirkende Teil des Außenumfangsmantels der Schnittstelleneinheit 34 ist in Figur 1a mit 46 bezeichnet. Die Ringdichtung 44 kann beispielsweise eine Lippendichtung oder ein O-Ring sein. Es versteht sich, dass alternativ eine solche Ringdichtung an der Schnittstelleneinheit 34 vorgesehen sein kann. Es ist darüber hinaus vorstellbar, auf ein gesondertes Dichtelement zu verzichten, sofern die Außenoberfläche der Schnittstelleneinheit 34 und die Innenoberfläche des Einführtrichters 20 hinreichend glatt sind und hinreichend eng aneinander zu liegen kommen.

Analog der Ringdichtung 44 ist eine weitere Ringdichtung 52 an der Saugringeinheit 16 in Richtung auf das Auge versetzt angeordnet. Für die weitere Ringdichtung 52 gilt hinsichtlich Ausgestaltung und ihrer Anordnung an der Saugringeinheit bzw. an der Schnittstelleneinheit 34 das vorstehend zur Ringdichtung 44 Gesagte entsprechend.

Die beiden vorstehend beschriebenen Ringdichtungen 44, 52 bilden dann, wenn die Schnittstelleinheit 34 an die Saugringeinheit 16 angekoppelt ist, einen Raum 58, vgl. Fig. 1b und 1c. Der Raum 58 wird vertikal oben und unten von den Dichtungen 44 und 52 begrenzt und auf beiden horizontalen Seiten einerseits von einer Außenfläche der Schnittstelleneinheit 34 als Dichtfläche 46 und andererseits einer Außenfläche 60 des Einführtrichters 20 der Saugringeinheit 16 als Dichtfläche 60. Der Raum 58 wird also ausschließlich durch die vorstehend genannten Elemente abgegrenzt und hat an keiner Stelle eine Berührung mit einer Komponente des Auges 10. Insbesondere besteht keine luftleitende Verbindung zwischen dem inneren des Raumes 58 und einer Oberfläche des Auges 10.

Gemäß Fig. 1b wird die Schnittstelleneinheit 34 in zumindest annähernd koaxialer Stellung in Bezug auf die Saugringeinheit 16 vertikal, d.h. in den Figuren nach unten abgesenkt. Dabei passen sich die Stellungen von Saugringeinheit 16 und Schnittstelleinheit 34 aufgrund der schwimmenden Lagerung letzterer (siehe unten) von selbst aneinander an.

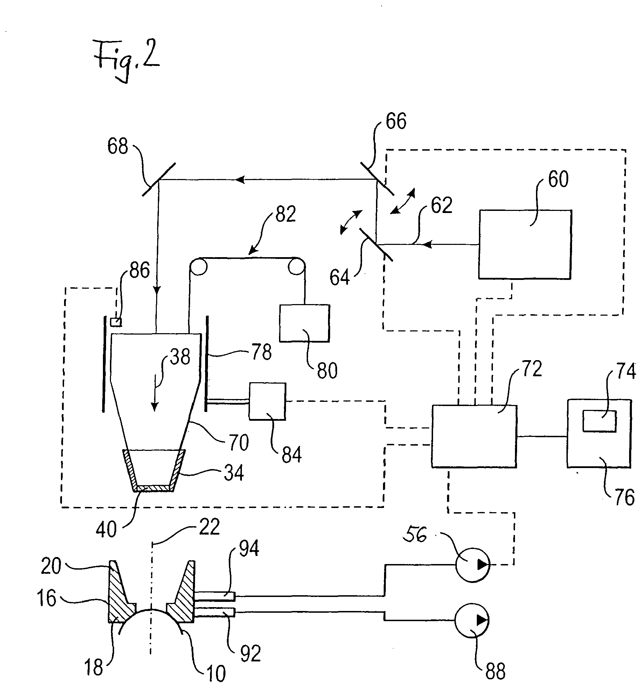

Die Absenkung der Schnittstelleneinheit in Pfeilrichtung 38 (entsprechend der Axialrichtung der Saugringeinheit 16) stoppt an einer vorgegebenen axialen Relativposition der beiden Einheiten, in welcher der Raum 58 hinreichend luftdicht abgeschlossen ist, um ihn über einen Evakuierungskanal 54 mittels einer Pumpe 56 (nicht gezeigt) zu evakuieren und so die Schnittstelleeinheit 34 fest an die Saugringeinheit 16 anzukoppeln. Dieser Zustand ist in Fig. 1c gezeigt. Um die Endstellung zu detektieren und damit die Absenkung zu beenden, kann an geeigneter Stelle ein Sensor angeordnet werden. Zum Beispiel kann ein Sensor 50 an der in den Figuren angegebenen Stelle positioniert sein. Es ist auch möglich, einen Sensor an der Fokussieroptik 70 (Fig. 2) anzuordnen. Es ist auch möglich, einen mechanischen Endschalter 86 (Fig. 2) vorzusehen. Der Sensor 50 ist geeignet positioniert, um dann eine entsprechendes Sensorsignal abzugeben, wenn die Relativstellung zwischen Schnittstelleneinheit und Saugringeinheit einen vorgegebenen Abstand der beiden Einheiten erreicht hat.

Fig. 2 zeigt schematisch Komponenten einer Vorrichtung zum Schneiden eines Gewebeteils eines Auges mittels Laserstrahlung, bei der die zuvor anhand der Fig. 1a-1c beschriebene Kopplung zwischen einer Saugringeinheit und einer Schnittstelleneinheit einsetzbar ist. Gleiche oder gleichwirkende Komponenten wie in den Figuren 1a-1c sind dabei mit gleichen Bezugszeichen bezeichnet. Zur Vermeidung von Wiederholungen wird auf die vorangehenden Ausführungen zu diesen Komponenten verwiesen.

Die Lasereinrichtung gemäß Fig. 2 umfasst eine Laserquelle 60 für gepulste Laserstrahlung mit Pulsdauern im Femtosekundenbereich. Der von der Laserquelle 60 abgegebene Laserstrahl - mit 62 bezeichnet - gelangt über eine hier von zwei steuerbaren Ablenkspiegeln 64, 66 gebildete Ablenkeinrichtung (Scanner) zu einem Umlenkspiegel 68, von welchem der Laserstrahl 62 in eine Fokussieroptik 70 gelangt. Am distalen, d.h. augennahen Ende der Fokussieroptik 70 ist die Schnittstelleneinheit 34 lösbar angekoppelt. Die Ablenkspiegel 64, 66 sind jeweils kippbar angeordnet und gestatten eine Ablenkung des Laserstrahls 62 in einer zur Strahllängsrichtung (z-Richtung) normalen x-y-Ebene. Sie werden von einer elektronischen Steuereinrichtung 72 nach Maßgabe eines durch Form und Lage des gewünschten Schnitts gegebenen Schnittprofils gesteuert. Das Schnittprofil ist in einem Steuerprogramm 74 verkörpert, welches in einem für die Steuereinrichtung 72 zugänglichen Speicher 76 abgelegt ist. Zur z-Verstellung des Strahlfokus kann entweder die Fokussieroptik 70 oder zumindest eine darin enthaltene Linse in Strahllängsrichtung unter der Steuerung der Steuereinrichtung 72 justierbar sein. Alternativ ist es möglich, eine Linse einer in Fig. 2 nicht näher dargestellten, zwischen der Laserquelle 60 und den Ablenkspiegeln 64, 66 angeordneten Strahlaufweitungsoptik in Strahllängsrichtung verstellbar anzuordnen, insbesondere eine eingangsseitige Zerstreuungslinse einer solchen Strahlaufweitungsoptik.

Die Fokussieroptik 70 ist an einer Halterung 78 gewichtskompensiert aufgehängt. Die Halterung 78 ist in Fig. 2 stark schematisch durch zwei beidseits der Fokussieroptik 70 gezeichnete senkrechte Striche angedeutet. Die Gewichtskompensation der Fokussieroptik 70 ist schematisch durch ein Gegengewicht 80 angedeutet, das über eine Seil-/Rollen-Anordnung 82 mit der Fokussieroptik 70 verbunden ist und eine deren Gewichtskraft kompensierende Gegenkraft auf die Fokussieroptik 70 ausübt. Eine Seil-/Rollen-Anordnung ist selbstverständlich nur ein Beispiel für die Anbindung eines Gegengewichts an die Fokussieroptik. Alternativ könnte beispielsweise ein Hebelsystem verwendet werden. Eine weitere mögliche Ausgestaltung ist in US 5,336,215 gezeigt, wo ein Federsystem zur Aufhängung einer Fokussieroptik verwendet wird.

Die Fokussieroptik 70 ist zusammen mit der Halterung 78 durch eine motorische, vorzugsweise elektromotorische Antriebseinheit 84 in vertikaler Richtung in den Einführtrichter 20 der auf dem Auge 10 sitzenden Saugringeinheit 16 absenkbar, wie durch den Richtungspfeil 38 angedeutet. Dabei ist die Fokussieroptik 70 nicht starr mit der Halterung 78 verbunden, sondern besitzt gegenüber der Halterung 78 eine gewisse Auslenkbarkeit entgegen der Absenkrichtung 38 nach oben. Wegen der Gewichtskompensation der Fokussieroptik 70 ist eine Auslenkung derselben gegenüber der Halterung bereits durch eine äußerst geringe Krafteinwirkung möglich. Der Moment, in dem die Applanationsplatte 40 in Kontakt mit dem Auge gelangt und einen Gegendruck vom Auge erfährt, kann deshalb schon zu einer Auslenkung der Fokussieroptik 70 relativ zu der Halterung 78 führen. Diese Auslenkung wird mittels eines relativ zu der Halterung 78 ortsfest angebrachten Endschalters 86 (alternativ z.B. Gegenkraftschalter) detektiert, der sein Schaltsignal an die Steuereinrichtung 72 liefert. Das Schalten des Endschalters 86 signalisiert der Steuereinrichtung 72 somit das Erreichen der vorgegebenen Relativstellung zwischen Schnittstelleneinheit 34 und Saugringeinheit 16 (z.B. Berühren des Einführtrichters 20 durch die Schnittstelleneinheit 34), in der die weitere motorische Absenkbewegung der Fokussieroptik 70 zu stoppen ist. Dementsprechend steuert die Steuereinrichtung 72 bei Erhalt des Schaltsignals von dem Endschalter 86 die Antriebseinheit 84 im Sinne eines Betriebsstopps. Das vorherige Absenken der Fokussieroptik 70 durch die Antriebseinheit 84 kann ebenfalls von der Steuereinrichtung 72 nach Maßgabe des Steuerprogramms 74 gesteuert sein; alternativ ist es vorstellbar, dass der Bediener die Absenkbewegung mittels eines an die Steuereinrichtung 72 angeschlossenen Steuerknüppels händisch initialisiert, wobei bei Erreichen der erwähnten vorgegebenen Relativposition zwischen Schnittstelleneinheit 34 und Saugringeinheit 16 die Steuereinrichtung 72 den Vorrang des Steuerknüppels aufhebt und selbsttätig den Betrieb der Antriebseinheit 84 stoppt.

Wegen des Vorhandenseins des Endschalters 86 ist in diesem Fall ein an den Komponenten 16 oder/und 34 angebrachter Sensor verzichtbar.

Die Lasereinrichtung gemäß Fig. 2 umfasst darüber hinaus zwei Evakuierungspumpen 88, 56, welche über geeignete Schlauchleitungen an je einen an der Saugringeinheit 16 ausgebildeten Anschlussstutzen 92 bzw. 94 angeschlossen sind. In den Anschlussstutzen 92 mündet der Evakuierungskanal 28 gemäß den Fig. 1a-1c; die Evakuierungspumpe 88 dient somit zur Evakuierung der Saugkammer 30. Dagegen mündet in den Anschlussstutzen 94 der Evakuierungskanal 54, weswegen die Evakuierungspumpe 56 zur Evakuierung des Raums 58 der Figuren 1a-1c dient.

Bei zuvor bereits evakuierter Saugkammer 30 wird in der zuvor beschriebenen Koppelstellung von Schnittstelleneinheit 34 und Saugringeinheit 16 die Evakuierungspumpe 56 betätigt, um den Raum 58 zu evakuieren. Diese Evakuierung des Raums 58 kann automatisch aufgrund der mit dem Sensor 50 detektierten Relativposition der beiden Einheiten durch die Steuereinrichtung 72 eingeleitet werden, oder auch willkürlich von Hand durch den Arzt. Es ist auch möglich, die Evakuierung des Raumes 58 mittels des Schalters 86 zu steuern.

## Patentansprüche

1. Vorrichtung zum Schneiden eines Gewebeteils eines Auges mittels Laserstrahlung, umfassend
- eine auf das Auge aufsetzbare Saugringeinheit (16) mit einer Ringachse (22), und
- eine von der Saugringeinheit (16) gesonderte, längs der Ringachse (22) in Koppelkontakt mit dieser bewegbare mechanischen Schnittstelleneinheit (34), die mit optischen Mitteln (70) mechanisch koppelbar ist, welche die Laserstrahlung auf oder in das Gewebeteil (12) des Auges fokussieren,
**gekennzeichnet durch**
- Dichtmittel (44, 52), die bei Bewegung der Schnittstelleneinheit (34) in Koppelkontakt mit der Saugringeinheit (16) einen evakuierbaren Raum (58) bilden, der von Dichtflächen (46, 60) der Schnittstelleneinheit (34) und der Saugringeinheit (16) und den Dichtmitteln (44, 52) abgegrenzt ist und an keiner Stelle eine Berührung mit einer Komponente des Auges hat, und
- Eine Pumpe (56) und einen Evakuierungskanal (54) zum Evakuieren des genannten Raumes (58).

2. Vorrichtung nach Anspruch 1, **dadurch gekennzeichnet, dass** die Dichtflächen (46, 60) zumindest im Bereich der Dichtmittel (44, 52) kegelstumpfförmig sind.

## Claims

1. Apparatus for cutting a tissue part of an eye by means of laser radiation, comprising
- a suction ring unit (16) that can be placed onto the eye and has a ring axis (22), and
- a mechanical interface unit (34) that is separate from the suction ring unit (16) and movable with coupling contact with the latter along the ring axis (22), said interface unit being mechanically coupleable to optical means (70) which focus the laser radiation onto or into the tissue part (12) of the eye,
**characterized by**
- sealing means (44, 52) which form a space (58) that can be evacuated when the interface unit (34) moves with coupling contact with the suction ring unit (16), said evacuatable space being delimited by sealing faces (46, 60) of the interface unit (34) and the suction ring unit (16) and by the sealing means (44, 52) and said evacuatable space having no contact with a component of the eye at any location, and
- a pump (56) and an evacuation channel (54) for evacuating the aforementioned space (58).

2. Apparatus according to Claim 1, **characterized in that** the sealing faces (46, 60) have a conical frustum shape, at least in the region of the sealing means (44, 52).

## Revendications

1. Dispositif pour couper une pièce de tissu d'un oeil au moyen d'un rayonnement laser, comprenant
- une unité à ventouse (16) pouvant être posée sur l'oeil, ayant un axe de bague (22), et
- une unité d'interface (34) séparée de l'unité à ventouse (16), pouvant être déplacée mécaniquement avec celle-ci en contact d'accouplement le long de l'axe de bague (22), laquelle peut être accouplée mécaniquement à des moyens optiques (70) qui concentrent le rayonnement laser sur ou dans la partie de tissu (12) de l'oeil,
**caractérisé par**
- des moyens d'étanchéité (44, 52) qui, lors du mouvement de l'unité d'interface (34) en contact d'accouplement avec l'unité à ventouse (16), forment un espace (58) pouvant être mis sous vide, lequel est délimité par les surfaces d'étanchéité (46, 60) de l'unité d'interface (34) et de l'unité à ventouse (16) et les moyens d'étanchéité (44, 52) et ne se trouve en aucun point en contact avec un composant de l'oeil, et
- une pompe (56) et un canal de mise sous vide (54) destinés à la mise sous vide dudit espace (58).

2. Dispositif selon la revendication 1, **caractérisé en ce que** les surfaces d'étanchéité (46, 60) sont de forme tronconique au moins dans la zone des moyens d'étanchéité (44, 52).
